# DEMANDE DE BREVET EUROPEEN

(11) **EP 2 878 293 A1**
(43) Date de publication de la demande: **03.06.2015**
(21) Numéro de dépôt: 14193330.9
(22) Date de dépôt: 14.11.2014
(51) Int. Cl.: A61K 8/38, A61K 8/41, A61K 8/43, A61Q 5/08, A61K 8/19, A61K 8/20, A61K 8/22

(54) **Composition de décoloration de poils humains, procédé et kit de décoloration**

(30) Priorité: 22.11.2013 FR 1361518
(71) Demandeur: Berdoues Parfums et Cosmétiques, 31270 Cugnaux (FR)
(72) Inventeur: Coutelle, Hervé, 31860 Labarthe sur Lèze (FR)
(74) Mandataire: Cabinet BARRE LAFORGUE & associés

(57) **Abrégé**

L'invention concerne une composition sans ammoniaque et sans sel d'ammonium, cosmétiquement acceptable comprenant :
- au moins un agent de décoloration de poils, et ;
- au moins une amine organique choisie à l'exclusion de tout acide α-aminé basique, et ;
- au moins une base forte minérale ;
caractérisée en ce que ladite au moins une base forte minérale :
- présente dans la composition une proportion massique comprise entre 0,2 % et 2 %, et ;
- est dans la composition en une quantité adaptée pour que le pH de la composition soit compris entre pH 9 et pH 12.

## Description

L'invention concerne une composition de décoloration de poils d'au moins une partie de surface d'épiderme d'un être humain, un procédé de décoloration de poils utilisant une telle composition, et un nécessaire, aussi nommé "kit" pour la mise en oeuvre d'un tel procédé. L'invention vise donc une composition de décoloration de poils d'un être humain, d'éclaircissement de la couleur desdits poils et de dissimulation et d'estompage desdits poils pour les rendre quasi-invisibles sur l'épiderme d'un être humain à peau claire -notamment d'un être humain du type caucasien-.

L'invention trouve donc ses applications dans le domaine de la cosmétique dans lequel sont recherchées des solutions pour améliorer l'apparence et l'esthétique d'une personne.

On connait des compositions d'éclaircissement de fibres de kératine humaines contenant de l'ammoniaque. L'utilisation d'ammoniaque dans de telles compositions présente des inconvénients. Ces inconvénients résident dans l'odeur d'ammoniac dégagée par ladite composition d'éclaircissement. Cette odeur est incommodante pour la personne souhaitant améliorer son apparence et pour l'esthéticien(ne) prodiguant ce traitement. En outre, l'utilisation de telles compositions d'éclaircissement peut conduire, chez la personne souhaitant améliorer son apparence, à des réactions d'intolérance et/ou d'allergie susceptibles d'entrainer momentanément une irritation de la peau et l'apparition de rougeurs inesthétiques. L'utilisation d'ammoniaque dans une composition de décoloration de poils pose donc problème.

On connaît aussi de FR 2 940 080 une composition d'éclaircissement de fibres de kératine exempte d'ammoniaque obtenue par mélange extemporané d'une première composition anhydre formée de cire liquide de Jojoba ou d'huile de vaseline, de monolaurate de sorbitan oxyéthyléné (4OE), de mono-éthanolamine et d'arginine et d'une deuxième composition oxydante aqueuse comprenant du peroxyde d'hydrogène 20V (6 % en poids de peroxyde d'hydrogène). FR 2 940 080 explique que le fait de purement et simplement remplacer en totalité ou en partie l'ammoniaque par un ou plusieurs autre(s) agent(s) alcalin(s) classique(s) ne conduit pas à des compositions aussi efficaces que celles à base d'ammoniaque, notamment parce que ces agents alcalins ne conduisent pas à un éclaircissement suffisant des fibres de kératine en présence de l'agent oxydant.

On connait aussi de FR 2 940 080 un procédé de décoloration dans lequel, on applique la composition de décoloration sur une mèche de couleur châtain (hauteur de ton de 5) et on maintient la composition de décoloration sur ladite mèche pendant une durée (temps de pause) de 30 minutes avant son rinçage et son lavage au moyen d'un shampooing.

Un tel procédé de décoloration nécessite un temps de pause de longue durée et l'utilisation d'une composition de décoloration comprenant de l'arginine à titre d'acide aminé basique. Par conséquent, une telle composition de décoloration est complexe dans sa préparation.

L'invention vise à pallier ces inconvénients en proposant une composition de décoloration de poils humains, un procédé et un kit de décoloration qui ne présentent pas les inconvénients liés à l'utilisation d'ammoniaque.

L'invention vise également à proposer une composition de décoloration, un procédé de décoloration et un kit pour la décoloration de poils humains qui présentent une efficacité de décoloration comparable ou améliorée par rapport aux compositions, procédé et kits connus pour une durée de contact entre ladite composition de décoloration et les poils humains qui est réduite.

Un autre objectif de l'invention est de fournir une composition de décoloration, un procédé et un kit de décoloration de poils humains qui sont peu agressifs pour les poils humains.

L'invention vise de surcroît à proposer une composition de décoloration, un procédé de décoloration et un kit qui soient faciles à utiliser, et n'impliquent pour leur mise en oeuvre que peu de manipulations. À cet égard, l'invention vise en outre à proposer une composition de décoloration, un procédé de décoloration et un kit qui respectent les habitudes des utilisateurs/utilisatrices.

Pour ce faire, l'invention concerne une composition -notamment une composition de décoloration de poils- sans ammoniaque et sans sel d'ammonium, cosmétiquement acceptable comprenant :
- au moins un agent de décoloration de poils -notamment de poils humains-, et ;
- au moins une amine organique choisie à l'exclusion de tout acide α-aminé basique, et ;
- au moins une base forte minérale ;
caractérisée en ce que ladite au moins une base forte minérale :
- présente dans la composition une proportion massique comprise entre 0,2 % et 2 %, et ;
- est présente dans la composition en une quantité adaptée pour que le pH de la composition soit compris entre pH 9,0 et pH 12, notamment compris entre pH 9,5 et pH 12,0, de préférence compris entre pH 9,5 et pH 11,0.

L'invention vise donc une composition, dite composition de décoloration, qui est prête à l'emploi. Une telle composition de décoloration est préparée extemporanément pour pouvoir être appliquée sur au moins une partie d'épiderme -notamment sur au moins une partie d'épiderme humain- présentant des poils à décolorer.

Avantageusement et selon l'invention, la composition de décoloration est une composition aqueuse.

Dans toute la suite, l'expression "cosmétiquement acceptable" précise que la composition de décoloration et les compositions pour l'obtention d'une telle composition de décoloration sont aptes à pouvoir être appliquées sur au moins une partie d'épiderme d'un être vivant -notamment d'un être humain- en vue de son traitement cosmétique. Une telle composition de décoloration peut donc comprendre par exemple des excipients cosmétiquement acceptables, des agents conservateurs cosmétiquement acceptables, des gélifiants cosmétiquement acceptables, des émulsionnants cosmétiquement acceptables et/ou des parfums cosmétiquement acceptables.

On qualifie de "organique" tout composé ou groupement constitués d'atomes de carbone et d'atomes d'hydrogène et éventuellement d'atomes d'oxygène et/ou d'atomes d'azote liés entre eux par des liaisons covalentes. En particulier, on entend par "amine organique" un composé constitué d'atomes de carbone, d'atomes d'hydrogène, d'au moins un atome d'azote et éventuellement d'au moins un atome d'oxygène, lesdits atomes étant liés entre eux par des liaisons covalentes.

La composition de décoloration selon l'invention est une composition sans ammoniaque, c'est-à-dire exempte d'ammoniaque. Une telle composition de décoloration selon l'invention ne comprend ni ammoniac (NH₃) gazeux, ni ammoniaque (NH₄OH ou NH₃/H₂O) en solution aqueuse Elle ne produit donc ni désagrément ni nuisances olfactives liés à l'utilisation d'ammoniaque, ni d'irritation ou d'érythème disgracieux lié à une réaction allergique.

La composition de décoloration est exempte de tout sel(s) d'ammonium tels que, par exemple, d'acétate d'ammonium et de carbonate d'ammonium. Avantageusement et selon l'invention, la composition de décoloration selon l'invention est une composition sans sel(s) d'ammonium. Par "sel d'ammonium", on entend un composé formé d'un anion et d'un cation dans lequel le cation est le cation ammonium de formule NH₄⁺.

Les inventeurs ont observé qu'il est possible de mettre en contact une composition de décoloration selon l'invention sur au moins une partie d'épiderme d'une personne souhaitant bénéficier d'un soin esthétique de décoloration de poils disgracieux pendant une durée de contact qui est suffisamment courte pour décolorer lesdits poils sans les abimer de façon irréversible.

Ils ont ainsi observé que la combinaison d'au moins une amine organique et d'au moins une base forte permet une décoloration des poils qui est plus rapide que les décolorations effectuées avec les compositions de l'état de la technique. Une composition selon l'invention permet en fait :
- un gonflement des fibres de kératine constitutives des poils, un écartement des écailles de surface des poils, favorisant l'accès de l'agent de décoloration à l'intérieur des poils et au contact des pigments -notamment de la mélanine- et leur oxydation, et ;
- simultanément, la décomposition d'au moins un agent de décoloration dans la composition de décoloration, l'oxydation des pigments -notamment de la mélanine- et leur décoloration partielle ou totale.

Avantageusement, la composition de décoloration est une composition cosmétiquement acceptable susceptible de pouvoir être appliquée sur au moins une partie de surface d'épiderme -notamment sur au moins une partie de surface d'épiderme humain- présentant des poils à dépigmenter et est adaptée pour pouvoir résider au contact desdits poils pendant une durée suffisante pour entrainer leur décoloration.

Avantageusement et selon l'invention, la composition de décoloration est une composition aqueuse. Elle comprend donc de l'eau. Il n'est cependant pas exclu que la composition de décoloration comprenne aussi au moins un solvant autre que de l'eau.

Avantageusement et selon l'invention, la composition de décoloration est une composition aqueuse liquide. Avantageusement, la composition de décoloration peut aussi être sous forme d'une solution colloïdale -notamment d'une solution colloïdale aqueuse- ou d'une émulsion aqueuse. Avantageusement et selon l'invention, la composition de décoloration peut être sous forme d'un gel cosmétiquement acceptable. Avantageusement et selon l'invention, la composition de décoloration peut être sous forme d'une mousse cosmétiquement acceptable. Une telle solution colloïdale, une telle émulsion, un tel gel et une telle mousse sont adaptés pour pouvoir être appliqués sur l'épiderme et résider au contact des poils à décolorer pendant une durée suffisante pour permettre leur décoloration.

Avantageusement et selon l'invention, la composition de décoloration est exempte d'acides α-aminés basiques -notamment d'arginine (en particulier de L-arginine), de lysine (en particulier de L-lysine) et d'histidine (en particulier de L-histidine). Avantageusement, une composition de décoloration selon l'invention exempte d'ammoniaque, exempte de sels d'ammonium et exempte d'acides α-aminés basiques mais comprenant au moins une amine organique (à l'exclusion de tout acide α-aminé basique) et au moins une base forte minérale, permet l'obtention d'une décoloration des poils avec un temps réduit de contact (temps de pause) de la composition de décoloration avec les poils humains. Ainsi, il n'est pas nécessaire de préparer une composition de décoloration comprenant des acides α-aminés -notamment d'acides α-aminés basiques- de restauration de la structure de la cuticule des poils humains ayant bénéficié d'un traitement de décoloration. Une telle composition de décoloration selon l'invention est donc une composition de décoloration qui est simplifiée par rapport aux compositions de décoloration de l'état de la technique.

Avantageusement et selon l'invention, la composition de décoloration est exempte d'acide α-aminé. La composition de décoloration est exempte de tout acide α-aminé choisi dans le groupe formé de la L-alanine, de la L-arginine, de la L-asparagine, de l'acide L-aspartique, de la L-cystéine, de l'acide L-glutamique, de la L-glutamine, de la L-glycine, de la L-histidine, de la L-isoleucine, de la L-leucine, de la L-lysine de la L-méthionine, de la L-phénylalanine, de la L-proline, de la L-pyrrolysine, de la L-sélénocystéine, de la L-sérine, de la L-thréonine, du L-tryptophane, de la L-tyrosine et de la L-valine.

Avantageusement et selon l'invention, la composition de décoloration comprend au moins un agent de décoloration de poils apte à se décomposer et à libérer un agent d'oxydation d'un pigment de poils, notamment de la mélanine sous l'effet de ladite composition de décoloration.

Avantageusement et selon l'invention, chaque agent de décoloration est choisi dans le groupe formé du peroxyde d'hydrogène (H₂O₂), du peroxyde d'hydrogène combiné à l'urée (CH₄N₂O/H₂O₂) -aussi appelé peroxyde de carbamide-, des bromates (BrO₃⁻) d'un métal alcalin (par exemple du bromate de sodium, BrO₃Na ou du bromate de potassium, BrO₃K), des ferricyanures ((Fe(CN)₆)³⁻) d'au moins un métal alcalin (par exemple du ferricyanure de potassium, Fe(CN)₆K₃), des sels peroxygénés -tels que les persulfates ((S₂O₈)²⁻) d'au moins un métal alcalin (par exemple du persulfate de sodium S₂O₈Na₂ et du persulfate de potassium S₂O₈K₂), les persulfates ((S₂O₈)²⁻) d'un métal alcalino-terreux (par exemple du persulfate de magnésium S₂O₈Mg, du persulfate de strontium S₂O₈Sr et du persulfate de calcium S₂O₈Ca), les perborates ((B₂O₄(OH)₄)²⁻) d'au moins un métal alcalin (par exemple du perborate de sodium B₂O₄(OH)₄)Na₂ et du perborate de potassium B₂O₄(OH)₄)K₂), les perborates ((B₂O₄(OH)₄)²⁻) d'un métal alcalino-terreux (par exemple du perborate de magnésium B₂O₄(OH)₄)Mg, du perborate de strontium B₂O₄(OH)₄)Sr et du perborate de calcium B₂O₄(OH)₄)Ca), les percarbonates d'au moins un métal alcalin -par exemple le percarbonate de sodium (2Na₂CO₃·3H₂O₂)- les percarbonates d'un métal alcalino-terreux (par exemple du percarbonate de magnésium et du percarbonate de strontium) et les peracides, chacun des agents de décoloration étant utilisé seul ou en mélange d'une pluralité d'agents de décoloration dans la composition.

Dans un mode de réalisation particulier, avantageusement et selon l'invention, l'agent de décoloration étant le peroxyde d'hydrogène (H₂O₂), sa proportion massique dans la composition est comprise entre 0,1 % et 12 % - notamment comprise entre 0,5 % et 10 %, de préférence entre 1 % et 10 %.

Une telle composition de décoloration comprend donc une quantité de peroxyde d'hydrogène (H₂O₂) apte à libérer de l'oxygène moléculaire (O₂) dans une quantité volumique comprise entre 0,3 L (dite solution 0,3V) et 40 L (dite solution 40V) -notamment comprise entre 3,25 L (dite solution 3,25V) et 34V (dite solution 34V), de préférence comprise entre 3,25 L et 17 L (dite solution 17V) par litre de composition de décoloration. Une telle composition de décoloration peut comprendre une quantité de peroxyde d'hydrogène (H₂O₂) apte à libérer de l'oxygène moléculaire (O₂) dans une quantité volumique comprise entre 5 L (dite solution 5V, proportion massique 1,5 %) et 30 L (dite solution 30V, proportion massique de l'ordre de 9 %)- par litre de composition de décoloration. Dans tout le texte, on indique la concentration en volume (V) en peroxyde d'hydrogène d'une solution d'eau oxygénée par la valeur du volume d'oxygène moléculaire susceptible de pouvoir être libéré par 1 litre de ladite solution d'eau oxygénée.

Avantageusement et selon l'invention, au moins une amine organique est choisie dans le groupe formé des amines organiques présentant de 1 à 6 atomes de carbone -notamment de 2 à 6 atomes de carbone-. Avantageusement et selon l'invention, chaque amine organique est choisie dans le groupe formé des amines organiques présentant de 1 à 6 atomes de carbone -notamment de 2 à 6 atomes de carbone-.

Avantageusement et selon l'invention, au moins une amine organique est choisie dans le groupe formé des amines organiques aliphatiques.

Avantageusement, chaque amine organique est choisie dans le groupe formé des amines organiques aliphatiques. La composition de décoloration est exempte d'amine organique aromatique.

Au moins une amine organique de la composition de décoloration peut être une amine primaire (c'est-à-dire une amine organique de formule R-NH₂ dans laquelle R est un groupement organique) ou une amine organique secondaire (de formule générale R₁R₂NH ou R₁R₂NH₂⁺ dans lesquelles R₁ et R₂ sont des groupements organiques) ou une amine organique tertiaire (de formule générale R₃R₄R₅N ou R₃R₄R₅NH⁺ dans lesquelles R₃, R₄ et R₅ sont des groupements organiques).

Une composition de décoloration selon l'invention est exempte d'amine organique quaternaire et de sel d'ammonium quaternaire.

Avantageusement et selon l'invention, au moins une amine organique est une amine primaire hydrocarbonée, c'est-à-dire une amine organique de formule R'-NH₂ dans laquelle R' est un groupement constitué uniquement d'atomes de carbone (C) et d'atomes d'hydrogène (H). Avantageusement et selon l'invention, la composition de décoloration comprend de l'éthylamine (NH₂-CH₂-CH₃) à titre d'amine organique.

Avantageusement et selon l'invention, au moins une amine organique est une amine hydroxylée. Avantageusement et selon l'invention, au moins une amine organique est une amine primaire hydroxylée. Avantageusement et selon l'invention, elle est choisie dans le groupe formé de la mono-éthanolamine (NH₂-CH₂-CH₂-OH) et la mono-isopropanolamine (1-amino-2-propanol, NH₂-CH₂-CHOH-CH₃).

Avantageusement et selon l'invention, au moins une amine organique est une amine organique secondaire hydroxylée. Avantageusement et selon l'invention, elle est choisie dans le groupe formé de la di-éthanolamine (NH[-CH₂-CH₂-OH]₂) et de la di-isopropanolamine (NH[-CH₂-CHOH-CH₃]₂).

Avantageusement et selon l'invention, au moins une amine organique est choisie dans le groupe formé de la mono-éthanolamine de formule NH₂-CH₂-CH₂-OH/NH₃⁺-CH₂-CH₂-OH, de la mono-isopropanolamine de formule NH₂-CH₂-CHOH-CH₃/NH₃⁺-CH₂-CHOH-CH₃ et de l'éthylamine de formule NH₂-CH₂-CH₃/NH₃⁺-CH₂-CH₃ et de leurs sels.

Avantageusement et selon l'invention, au moins une amine organique est choisie dans le groupe formé des amines organiques tertiaires hydroxylées, notamment de la tri-éthanolamine (N[-CH₂-CH₂-OH]₃)-Avantageusement et selon l'invention, au moins une amine organique est choisie dans le groupe formé de la guanidine ([H₂N-]₂C=NH₂), de ses sels -notamment du carbonate de guanidine- et du bicarbonate d'amino-guanidine.

Avantageusement et selon l'invention, au moins une amine organique -notamment chaque amine organique- de la composition de décoloration est choisie dans le groupe formé de la mono-éthanolamine, de la mono-isopropanolamine, de l'éthylamine et de leurs sels, de la guanidine ([H₂N-]₂C=NH₂) et de ses sels -notamment du carbonate de guanidine-, du bicarbonate d'amino-guanidine et de la tri-éthanolamine (N[-CH₂-CH₂-OH]₃). Avantageusement, chaque amine organique est choisie dans le groupe formé de la mono-éthanolamine, de la mono-isopropanolamine, de l'éthylamine et de leurs sels, de la guanidine ([H₂N-]₂C=NH₂) et de ses sels -notamment du carbonate de guanidine-, du bicarbonate d'amino-guanidine et de la tri-éthanolamine (N[-CH₂-CH₂-OH]₃).

Avantageusement et selon l'invention, au moins une amine organique présente une proportion massique comprise entre 0,1 % et 40 % -notamment comprise entre 0,1 % et 20 %, de préférence comprise entre 0,5 % et 10 %, plus préférentiellement comprise entre 0,5 % et 3 %, en particulier sensiblement de l'ordre de 2 %- dans la composition. Avantageusement et selon l'invention, au moins une amine organique présente une proportion massique comprise entre 1,8 % et 2,2 % dans la composition.

Avantageusement et selon l'invention, au moins une base forte minérale est choisie dans le groupe formé de l'hydroxyde de sodium (NaOH), de l'hydroxyde de potassium (KOH) de l'hydroxyde de strontium (Sr(OH)₂), de l'hydroxyde de calcium (Ca(OH)₂) et de l'hydroxyde de lithium (LiOH).

Avantageusement et selon l'invention, au moins une base forte minérale présente une proportion massique comprise entre 0,2 % et 2 % dans la composition de décoloration. Avantageusement et selon l'invention, au moins une base forte minérale présente une proportion massique comprise entre 0,2 % et 1,3 %. Avantageusement et selon l'invention, au moins une base forte minérale présente une proportion massique comprise entre 0,2 % et 1 % notamment de l'ordre de 0,5%.

Avantageusement et selon l'invention, au moins une base forte minérale est présente dans la composition de décoloration en une quantité adaptée pour que le pH de la composition de décoloration soit compris entre pH 9 et pH 12, notamment compris entre pH 9 et pH 11,5, préférentiellement compris entre pH 9,5 et pH 11,5, plus préférentiellement compris entre pH 9,5 et pH 11 et pour permettre la décomposition d'au moins un agent de décoloration et la libération d'au moins un agent d'oxydation d'un pigment de poil dans la composition de décoloration.

Avantageusement et selon l'invention, la composition de décoloration comprend au moins un agent de renfort de la décoloration choisi dans le groupe formé de l'urée, des glycols tels que le glycérol, le propylène glycol (propane-1,2-diol), les di-propylène glycols, le pentylène glycol et les persulfates. Un tel agent de renfort de la coloration est bien entendu exempt d'ammoniaque. Il est aussi exempt de tout sel d'ammonium, de tout acide α-aminé basique. Un tel agent de renfort de la coloration est distinct de la base forte minérale et de l'amine organique. Dans ce premier mode de réalisation, l'agent de renfort de la décoloration est choisi pour favoriser la décomposition de l'agent de décoloration et la libération de l'agent d'oxydation dans la composition de décoloration, le gonflement des fibres de kératine et l'ouverture des écailles des poils et/ou du duvet, ladite ouverture favorisant la pénétration de l'agent de décoloration et/ou de l'agent oxydant au coeur des poils et/ou du duvet et leur décoloration. Dans ce premier mode de réalisation, le(s) agent(s) de renfort sont présents dans la composition de décoloration en proportion massique comprise entre 0,1 % et 40 % - notamment entre 0,5 % et 20 %-.

Avantageusement et selon l'invention, la composition de décoloration comprend au moins un solvant organique choisi dans le groupe formé des alcools linéaires comprenant de 2 à 4 atomes de carbone -notamment de l'éthanol-, des alcools ramifiés comprenant de 2 à 4 atomes de carbone -notamment de l'iso-propanol-, du di-éthyle-éther (CH₃-CH₂-O-CH₂-CH₃), du mono-éthyle-éther du di-éthylène glycol (CH₃-CH₂-O-CH₂-CH₂-OH) et des alcools aromatiques -notamment l'alcool benzylique et du phénoxy-éthanol. Dans ce deuxième mode de réalisation de l'invention, le(s) solvant(s) organique(s) sont présents dans la composition de dépigmentation en proportion massique comprise entre 0,05 % et 20 % -notamment entre 0,1 % et 10 %-.

Avantageusement et selon l'invention, la composition de décoloration comprend au moins un composé tensioactif de stabilisation de la composition de décoloration.

De tels composés tensioactifs peuvent être des composés tensioactifs non ioniques. Ils peuvent en particulier être choisis dans le groupe formé des tensioactifs non ioniques -notamment des alcools gras mono-alcoxylés, des alcools gras poly- alcoxylés, des alcools gras mono-glycéroxylés, des alcools gras poly-glycéroxylés et des alkyl-phénols alcoxylés. À titre d'exemples non limitatifs, le composé tensioactif non ionique est choisi dans le groupe formé :
- des alcools gras alcoxylés présentant de 8 à 30 atomes de carbone, lesdits alcools gras étant choisis dans le groupe formé des alcools gras saturés, des alcools gras insaturés, des alcools gras linéaires et des alcools gras ramifiés ;
- des amides grasses alcoxylées présentant de 8 à 30 atomes de carbone, lesdites amides grasses étant choisies dans le groupe formé des amides grasses saturées, des amides grasses insaturées, des amides grasses linéaires et des amides grasses ramifiées ;
- des esters d'un acide gras présentant de 8 à 30 atomes de carbone et de poly-éthylène-glycol, ledit ester d'acide gras étant choisi dans le groupe formé des esters d'acides gras saturés, des esters d'acides gras insaturés, des esters d'acides gras linéaires et des esters d'acides gras ramifiées ;
- des esters d'un acide gras présentant de 8 à 30 atomes de carbone et de sorbitol polyalcoxylé, ledit ester d'acide gras étant choisi dans le groupe formé des esters d'acides gras saturés, des esters d'acides gras insaturés, des esters d'acides gras linéaires et des esters d'acides gras ramifiés ;
- des huiles végétales éthoxylées saturées ou insaturées ;
- des polymères de l'oxyde d'éthylène et de l'oxyde de propylène, et ;
- un mélange d'au moins deux de ces composés tensioactifs.

Avantageusement, la proportion massique du(des) composé(s) tensioactif(s) dans la composition de décoloration est comprise entre 0,25 % et 7,5 %.

Rien n'empêche cependant de choisir un composé tensioactif dans le groupe formé des composés tensioactifs zwiterioniques. Rien n'empêche non plus de choisir un composé tensioactif dans le groupe formé des composés tensioactifs anioniques et des composés tensioactifs cationiques.

Avantageusement et selon l'invention, la composition sans ammoniaque et sans sel d'ammonium, cosmétiquement acceptable comprend -notamment est constituée de- :
- du peroxyde d'hydrogène, et ;
- au moins une amine organique choisie dans le groupe formé de la mono-éthanolamine, de la mono-isopropanolamine et de l'éthylamine et de leurs sels, et ;
- au moins une base forte minérale choisie dans le groupe formé de l'hydroxyde de sodium et de l'hydroxyde de potassium, et ;
- au moins un excipient cosmétiquement acceptable et exempt d'acide α-aminé basique, et ;
- de l'eau.

L'invention vise aussi un procédé de décoloration de poils -notamment de poils humains- dans lequel :
- on prépare extemporanément une composition de décoloration sans ammoniaque et sans sel d'ammonium, cosmétiquement acceptable comprenant :
   o au moins un agent de décoloration des poils ;
   o au moins une amine organique choisie à l'exclusion de tout acide α-aminé basique -notamment de tout acide α-aminé-, et ;
   o au moins une base forte minérale, puis ;
- on applique ladite composition de décoloration sur les poils à décolorer et on maintient ladite composition de décoloration en contact avec lesdits poils pendant une durée suffisante pour obtenir ladite décoloration ;
caractérisé en ce que ladite au moins une base forte minérale :
- présente dans la composition de décoloration une proportion massique comprise entre 0,2 % et 2 %, et ;
- est présente dans la composition de décoloration en une quantité adaptée pour que le pH de la composition de décoloration soit compris entre pH 9,0 et pH 12,0.

Un tel procédé de décoloration de poils humains dans lequel on utilise une composition de décoloration sans ammoniaque et sans sel d'ammonium selon l'invention est susceptible de pouvoir être mis en oeuvre sur au moins une partie pileuse du visage humain -notamment au niveau de la moustache se développant sur la lèvre supérieure, au niveau des sourcils et sur les joues- sur les avant-bras, sur les jambes, sur les aisselles, sur le pubis (pilosité du maillot), sur le buste et de façon générale sur toute surface pileuse du corps humain qu'une personne considère comme disgracieuse et souhaite atténuer et/ou rendre moins perceptible/visible. Il peut s'agir de poils colorés et rigides chez une personne adulte ou de duvet formé de poils naissants fins et souples et souvent de faible longueur. En tout état de cause, un tel procédé de décoloration ne vise pas la décoloration de cheveux qui ne sont pas des poils.

Avantageusement et selon l'invention, on prépare la composition de décoloration par mélange :
- d'une composition acide oxydante comprenant chaque agent de décoloration, et ;
- d'une composition alcaline comprenant chaque amine organique et chaque base forte minérale et présentant une valeur de pH comprise entre pH 10,0 et pH 13,0 -notamment comprise entre pH 12,0 et pH 13,0-.

Avantageusement et selon l'invention, la composition oxydante et la composition alcaline sont des compositions sans ammoniaque et sans sel d'ammonium et exemptes de tout acide α-aminé basique -en particulier exemptes de tout acide α-aminé-.

Avantageusement et selon l'invention, au moins l'une de la composition oxydante et de la composition alcaline est une composition liquide -notamment une composition aqueuse. Avantageusement, la composition oxydante et la composition alcaline sont des compositions liquides -notamment des compositions aqueuses-.

Avantageusement, on prépare la composition de décoloration par mélange d'un premier volume de composition oxydante et d'un deuxième volume de composition alcaline. Il est possible que, la composition oxydante et la composition alcaline étant des compositions liquides, le premier volume et le deuxième volume soient différents ou identiques.

Avantageusement et selon l'invention, au moins l'une des compositions oxydante et alcaline étant sous forme d'un solide à l'état divisé (poudre), on forme la composition de décoloration par mélange de la composition oxydante, de la composition alcaline et, le cas échéant d'une quantité prédéterminée d'au moins un solvant -notamment d'eau, en particulier d'eau déminéralisée.

Avantageusement et selon l'invention, la composition alcaline présente une proportion massique d'amine organique comprise entre 0,2 % et 50 % -de préférence comprise entre 0,5 % et 20 %-.

Avantageusement et selon l'invention, la composition alcaline comprend une proportion massique totale de base forte minérale comprise entre 0,2 % et 10 % -de préférence comprise entre 0,5 % et 10 %-.

Avantageusement et selon l'invention, la composition alcaline est choisie dans le groupe formé des gels alcalins, des solutions ou lotions -notamment des solutions ou lotions aqueuses- alcalines, des solutions colloïdales alcalines, des émulsions alcalines, des crèmes alcalines, des sticks alcalins et des solides -notamment des solides à l'état divisé- alcalins.

Avantageusement et selon l'invention, pour préparer la composition alcaline, on choisit un ordre d'addition des constituants de ladite composition alcaline dans lequel on introduit la(les) base(s) forte(s) minérale(s) préalablement à l'introduction de l'(des) amine(s) organique(s) en vue de former la composition alcaline. Bien qu'aucune explication ne soit donnée à ce phénomène, les inventeurs ont observé qu'une meilleure efficacité de décoloration -notamment un temps de pause réduit-(de 20 minutes à 5 minutes) est obtenu en introduisant d'abord la(les) base(s) forte(s) minérale(s), puis l'(les) amine(s) organique(s) pour former la composition alcaline. Les inventeurs ont en particulier observé qu'un tel ordre d'addition de la(des) base(s) forte(s) minérale(s) préalablement à l'addition de l'(des) amine(s) organique(s) en vue de former la composition alcaline permet d'initier la décoloration de poils au bout de 5 minutes de temps de pause et d'obtenir une décoloration optimale au bout de 10 minutes de temps de pause. En comparaison, l'addition de l'(des) amine(s) organique(s) préalablement à l'addition la(des) base(s) forte(s) minérale(s) en vue de former la composition alcaline ne permet d'initier la décoloration de poils qu'au bout de 20 minutes de temps de pause et d'obtenir une décoloration optimale au bout de 25 minutes de temps de pause.

Avantageusement et selon l'invention, la composition oxydante est choisie dans le groupe formé des gels oxydants, des solutions ou lotions -notamment des solutions ou lotions aqueuses- oxydantes, des solutions colloïdales oxydantes, des émulsions oxydantes, des crèmes oxydantes, des sticks oxydants et des solides -notamment des solides à l'état divisé- oxydants.

Avantageusement et selon l'invention, la composition oxydante comprend au moins une huile choisie dans le groupe formé des huiles végétales, en particulier des triesters du glycérol et d'acides gras saturés ou insaturés (triglycérides), des huiles minérales formées d'alcanes aromatiques ou aliphatiques, en particulier issues de la distillation du pétrole et des huiles de synthèse formées d'esters ou d'éthers linéaires ou ramifiés, aliphatiques ou aromatiques. L'huile végétale de la composition oxydante est de préférence choisie dans le groupe formé des huiles extraites de fruits, de fleurs, de graines, de feuilles, de tiges ou de racines de plantes. On choisit l'huile végétale de la composition oxydante de préférence dans le groupe formé des huiles d'amandes, des huiles d'avocat, des huiles de lin, des huiles de maïs, des huiles de blé, des huiles d'olive et des huiles de lotus, chacune de ces huiles pouvant être présente dans la composition oxydante seule ou en mélange avec d'autres huiles végétales, minérales ou de synthèse. L'huile minérale de la composition oxydante est préférentiellement choisie dans le groupe formé de l'huile de paraffine et de l'huile de vaseline. L'huile de synthèse de la composition oxydante est préférentiellement une huile de silicone.

Avantageusement et selon l'invention, la composition oxydante et la composition de décoloration comprennent au moins un agent épaississant choisi dans le groupe formé :
- des acides gras -notamment de l'acide stéarique, de l'acide palmitique, de l'acide oléique-,
- des esters d'un acide gras -notamment de l'acide stéarique, de l'acide palmitique, de l'acide oléique- et d'un alcool -notamment de glycol et de glycérol-, de préférence un mono-stéarate de glycérol ;
- des esters gras formés d'un acide carboxylique aliphatique présentant de 1 à 26 atomes de carbone et d'un alcool aliphatique présentant de 1 à 26 atomes de carbone, ledit acide carboxylique aliphatique étant choisi dans le groupe formé des acides carboxyliques aliphatiques saturés, des acides carboxyliques aliphatiques insaturés, des acides carboxyliques aliphatiques linéaires et des acides carboxyliques aliphatiques ramifiés et ledit alcool aliphatique étant choisi dans le groupe formé des acides carboxyliques aliphatiques saturés, des acides carboxyliques aliphatiques insaturés, des acides carboxyliques aliphatiques linéaires et des acides carboxyliques aliphatiques ramifiés. Avantageusement, on choisit l'ester gras dans le groupe formé des palmitates d'alkyle, tels que le palmitate d'éthyle, le palmitate d'isopropyle, le palmitate d'éthyl-2-hexyle, le palmitate d'octyle et des myristates d'alkyle, tels que le myristate d'isopropyle ;

- des cires végétales telles que la cire de carnauba, la cire de candelila ;
- des cires animales telles que la cire d'abeille, et ;
- des cires minérales et des cires synthétiques telles que les paraffines.

Avantageusement et selon l'invention, la composition oxydante et la composition de décoloration comprennent au moins un agent gélifiant choisi dans le groupe formé :
- des gélifiants cellulosiques ;
- des gélifiants obtenus par action d'un microorganisme tels que des gommes -notamment la gomme de xanthane (E415)- ;
- des silices précipitées ;
- des silices pyrogénées ;
- des polymères hydrosolubles synthétiques de l'acide acrylique, nommés "carbomer";
- des polymères amphiphiles comprenant une chaîne hydrocarbonée, et ;
- des polymères polyvinyliques.

Avantageusement et selon l'invention, la composition alcaline comprend au moins un tel agent gélifiant identique ou distinct de l'agent gélifiant de la composition d'oxydation, notamment choisi dans le groupe formé des polymères hydrosolubles synthétiques de l'acide acrylique, nommés "carbomer" et des gommes.

Avantageusement et selon l'invention, la composition oxydante comprend au moins un indicateur de mélange choisi dans le groupe formé des indicateurs de pH qui sont colorés à une valeur de pH comprise entre pH 8,0 et pH 12,0 et qui sont incolores à une valeur de pH comprise entre pH 1,0 et pH 8,0 et à une valeur de pH comprise entre pH 12 et pH 14. Avantageusement, la composition oxydante et la composition de décoloration comprennent au moins un indicateur de mélange choisi dans le groupe des phtaléines cosmétiquement acceptables, en particulier la O-crésolphtaléine.

Avantageusement et selon l'invention, la composition oxydante présente une valeur de pH inférieure à 7 -notamment comprise entre pH 1 et pH 5-. Une telle valeur de pH de la composition oxydante est adaptée pour permettre une stabilisation du peroxyde d'hydrogène dans la composition oxydante et pour permettre une libération de l'oxygène actif lors du mélange de la composition oxydante avec la composition alcaline. La composition oxydante comprend donc au moins un agent d'acidification de la composition oxydante choisi dans le groupe formé des acides forts minéraux -notamment l'acide phosphorique ou l'acide sulfurique- et des acides forts organiques -notamment l'acide benzoïque ou l'acide citrique-.

Avantageusement et selon l'invention, au moins un composé tensioactif présente dans la composition oxydante une proportion massique comprise entre 0,5 % et 15 %.

Avantageusement et selon l'invention, on maintient la composition de décoloration en contact avec les poils pendant une durée inférieure à 15 min -notamment comprise entre 3 min et 12 min, de préférence comprise entre 5 min et 10 min-.

Avantageusement et selon l'invention, on réalise chaque étape à température ambiante.

L'invention s'étend aussi à un nécessaire -ou kit- de décoloration -notamment un nécessaire -ou kit- de décoloration de poils- adapté pour préparer une composition de décoloration selon l'invention, comprenant :
- un premier dispositif contenant une composition oxydante acide sans ammoniaque et sans sel d'ammonium, cosmétiquement acceptable et comprenant au moins un agent de décoloration de poils, et ;
- un deuxième dispositif contenant une composition alcaline aussi sans ammoniaque et sans sel d'ammonium, cosmétiquement acceptable et comprenant au moins une amine organique et au moins une base forte minérale ;
caractérisé en ce que la composition oxydante et la composition alcaline sont exemptes de tout acide α-aminé basique, la composition alcaline présentant une valeur de pH comprise entre pH 10,0 et pH 13,0.

Avantageusement, les premier et deuxième dispositifs sont des flacons adaptés pour permettre un mélange de la composition oxydante avec la composition alcaline.

Avantageusement, dans une première variante d'un nécessaire selon l'invention, la composition oxydante est une composition aqueuse comprenant une proportion massique d'eau d'au moins 7 % -notamment d'au moins 20 % d'eau-. Une telle composition oxydante d'un nécessaire de décoloration selon cette première variante de l'invention est sous forme d'une émulsion ou d'un gel. Dans cette première variante d'un nécessaire selon l'invention, la composition oxydante comprend au moins un composé tensioactif.

Rien n'empêche cependant que dans une deuxième variante d'un nécessaire selon l'invention, la composition oxydante soit une composition sensiblement exempte d'eau.

Avantageusement et selon l'invention, la composition oxydante comprend aussi au moins un colorant et/ou au moins un pigment, et/ou au moins un agent humectant -notamment choisi dans le groupe formé du glycérol et de l'urée-, et/ou au moins un agent de conservation de la composition oxydante, et/ou au moins un parfum, par exemple choisi dans le groupe formé des huiles essentielles.

L'invention concerne également un procédé de décoloration, une composition de décoloration préparée lors de la mise en oeuvre d'un tel procédé et un nécessaire de décoloration caractérisés en combinaison par tout ou partie des caractéristiques mentionnées ci-dessus ou ci-après.

D'autres buts, caractéristiques et avantages de l'invention apparaîtront à la lecture de la description suivante et des exemples donnés uniquement à titre non limitatif.

Dans un procédé de décoloration de poils humains selon l'invention, on prépare une composition de décoloration par mélange extemporané d'une composition oxydante et d'une composition alcaline. On applique la composition de décoloration sur la partie d'épiderme de la personne souhaitant bénéficier de ce soin et présentant des poils à décolorer. On maintient ladite composition de décoloration sur les poils à décolorer pendant une durée suffisante pour permettre la décoloration des poils puis on rince à l'eau et on sèche les poils décolorés.

La composition oxydante est une composition liquide comprenant au moins un agent de décoloration des poils humains. En particulier, la composition oxydante comprend de l'eau oxygénée (H₂O₂) à titre d'agent de décoloration et de libération d'oxygène moléculaire actif sur l'oxydation de la mélanine des poils humains. On choisit une solution concentrée en eau oxygénée -par exemple une solution de peroxyde d'hydrogène 130V comprenant une proportion massique de l'ordre de 35 % de peroxyde d'hydrogène dans l'eau-. Il est possible d'obtenir une composition oxydante selon l'invention comprenant entre 0,1 % et 12 % (en volume) d'une solution concentrée en peroxyde d'hydrogène et de façon à former la composition oxydante comprenant du peroxyde d'hydrogène à une concentration comprise entre 0,1V (de l'ordre de 0,03 % en masse) et 20V (de l'ordre de 6 % en masse).

Une telle composition oxydante comprend en outre au moins un agent d'acidification de la composition oxydante et de stabilisation de l'agent de décoloration choisi dans le groupe formé des acides forts minéraux cosmétiquement acceptables et des acides forts organiques cosmétiquement acceptables et en quantité adaptée pour que le pH de la composition oxydante soit inférieur à 7 -notamment compris entre pH 1 et pH 5-.

Une telle composition oxydante comprend aussi au moins un gélifiant apte à permettre une adaptation de la consistance de la composition oxydante et de la composition de décoloration obtenue par mélange de ladite composition oxydante avec la composition alcaline. Il peut s'agir de tout type de gélifiant cosmétiquement acceptable, comme par exemple un gélifiant cellulosique, un gélifiant obtenus par action d'un microorganisme tel que la gomme de xanthane (E415), une silice précipitée, une silice pyrogénée, un polymère hydrosoluble synthétiques de l'acide acrylique (nommé "carbomer"), un polymère amphiphile comprenant une chaîne hydrocarbonée, un polymère polyvinylique.

Une telle composition d'oxydation peut aussi comprendre au moins un composé tensioactif, dit émulsionnant, de stabilisation de la composition d'oxydation. Un tel émulsionnant permet de former et stabiliser l'émulsion formée entre l'eau et l'huile choisie dans le groupe formé des huiles végétales, des huiles minérales et des huiles de synthèse. On choisit cet émulsionnant de façon à former, selon les cas, une émulsion, dite émulsion directe, d'une huile dans l'eau ou une émulsion, dite émulsion inverse, d'eau dans une huile.

Dans un premier mode de réalisation d'un procédé de dépigmentation de poils et/ou de duvet selon l'invention, on prépare une composition oxydante liquide comprenant de l'eau oxygénée (H₂O₂). Une telle composition oxydante est donnée au tableau 1 ci-après.

**Tableau 1**

| MATIÈRE PREMIÈRE | Proportion massique, % |
|---|---|
| CARBOMER | 0,05 - 5 |
| GOMME XANTHANE | 0,1 - 5 |
| EDTA DISODIQUE | 0,01 - 0,5 |
| ACIDE BENZOÏQUE | 0,01 - 5 |
| ÉMULSIONANT | 0,5 - 15 |
| ALCOOL ÉTHYLIQUE | 0,5 - 10 |
| INDICATEUR DE MÉLANGE | 0,001 - 1 |
| HUILE ESSENTIELLE DE CAMOMILLE | 0,005 - 0,5 |
| EXTRAIT LIPOPHILE DE LOTUS | 0,01 - 5 |
| PARFUM | 0,1 - 2 |
| ACIDE PHOSPHORIQUE | 0,005 - 1 |
| EAU OXYGÉNÉE 130 VOL | 0,1 - 12 |
| D-PANTHÉNOL | 0,05 - 2 |
| EAU DÉMINÉRALISÉE | QSP 100 % |

Une telle composition oxydante comprend en particulier des polymères synthétiques de l'acide acrylique, désignés sous le nom générique de "Carbomer", à titre d'agents épaississants et stabilisateurs de l'émulsion formée par mélange de la composition oxydante et de la composition alcaline.

La composition oxydante comprend en outre un indicateur de la qualité du mélange de la famille des phtaléines tel que décrit dans la demande de brevet FR 2 6106 324-. Lors du mélange de la composition oxydante (acide) et de la composition basique, l'indicateur incolore dans la composition oxydante acide prend une couleur rose révélatrice d'une valeur de pH comprise entre 8 et 12 dans la composition de traitement et attestant de la qualité du mélange de la composition oxydante et de la composition basique.

On prépare aussi une composition alcaline liquide comprenant de la mono-éthanolamine et de l'hydroxyde de sodium. Une telle composition alcaline est donnée au tableau 2 ci-après.

**Tableau 2**

| MATIÈRE PREMIÈRE | Proportion massique, % |
|---|---|
| CARBOMER | 0,05 - 5 |
| SALCARE^{®} SC 80 | 0,01 - 8 |
| MONO-ÉTHANOLAMINE | 0,5 - 20 |
| HYDROXYDE DE SODIUM, qsp pH 14 | 0,2 - 10 |
| EAU DÉMINÉRALISÉE | QSP 100 % |

Une telle composition alcaline comprend au moins un copolymère acrylique anionique formateur d'émulsion aqueuse (SALCARE® SC 80).

On forme la composition de décoloration prête à l'emploi selon l'invention par mélange à homogénéité d'une masse de composition oxydante et d'une même masse de composition alcaline, la composition de décoloration virant à la couleur rose lors de la complétion du mélange.

Dans un deuxième mode de réalisation d'un procédé de décoloration de poils humains selon l'invention, on prépare une composition oxydante liquide comprenant de l'eau oxygénée (H₂O₂). Une telle composition oxydante est donnée au tableau 3 ci-après.

**Tableau 3**

| MATIÈRE PREMIÈRE | Proportion massique, % |
|---|---|
| VITAMINE F | 0,01 - 5 |
| CARBOMER | 0,05 - 5 |
| GOMME XANTHANE | 0,1 - 5 |
| EDTA DISODIQUE | 0,01 - 0,5 |
| ACIDE BENZOÏQUE | 0,01 - 5 |
| EMULSIONANTS | 0,5 - 15 |
| ALCOOL ÉTHYLIQUE | 0,5 - 10 |
| INDICATEUR DE MÉLANGE | 0,001 - 1 |
| EXTRAIT LIPOPHILE DE CALENDULA | 0,01 - 5 |
| PARFUM | 0,1 - 2 |
| ACIDE PHOSPHORIQUE | 0,005 - 1 |
| EAU OXYGÉNÉE 130 VOL | 0,1 - 12 |
| EAU DÉMINÉRALISÉE | QSP 100 % |

On prépare aussi une composition alcaline liquide comprenant de la mono-isopropanolamine et de l'hydroxyde de potassium. Une telle composition alcaline est donnée au tableau 4 ci-après.

**Tableau 4**

| MATIÈRE PREMIÈRE | Proportion massique, % |
|---|---|
| CARBOMER | 0,05 - 5 |
| GOMME XANTHANE | 0,01 - 8 |
| EMULSIONANTS | 0,5 - 15 |
| ALCOOL ÉTHYLIQUE | 0,5 - 10 |
| MONO-ISOPROPANOLAMINE | 0,5 - 20 |
| HYDROXYDE DE POTASSIUM, qsp pH 13,5 | 0,2 - 10 |
| EAU DÉMINÉRALISÉE | QSP 100 % |

On forme la composition de décoloration prête à l'emploi selon l'invention par mélange à homogénéité d'une masse de composition oxydante et d'une même masse de composition alcaline, la composition de décoloration virant à la couleur rose lors de la complétion du mélange.

Dans un troisième mode de réalisation d'un procédé de dépigmentation de poils humains selon l'invention, on prépare une composition, dite composition oxydante, liquide comprenant de l'eau oxygénée (H₂O₂) identique à la composition oxydante donnée au tableau 3. On prépare aussi une composition alcaline liquide comprenant de la mono-isopropanolamine et de l'hydroxyde de potassium identique à la composition alcaline donnée au tableau 4.

On forme une composition de décoloration prête à l'emploi selon l'invention comprenant 49 % (en masse) de la composition alcaline (exemple 2), 49 % (en masse) de composition oxydante (exemple 2) et 2 % (en masse) d'une composition de renforcement comprenant entre 0,05 % et 15 % de bicarbonate de sodium.

On applique la composition de décoloration sur la partie de surface d'épiderme dont on souhaite décolorer les poils et on maintient ladite composition de décoloration pendant une durée adaptée pour permettre l'obtention d'un éclaircissement et/ou une décoloration desdits poils. On élimine la composition de décoloration et on rince à l'eau la partie de surface de peau dont les poils sont décolorés et quasiment non-distinguables de l'épiderme sous-jacent.

### EXEMPLE 1 - Composition de décoloration selon l'invention.

On prépare extemporanément une composition de décoloration selon l'invention par mélange à homogénéité d'une masse de composition alcaline et d'une même masse de composition oxydante. La constitution de la composition oxydante est donnée au tableau 5 ci-après.

**Tableau 5**

| MATIÈRE PREMIÈRE | Proportion massique, % |
|---|---|
| DEPILINE™ | 0,35 |
| CARBOPOL^{®} 980 | 0,40 |
| GOMME XANTHANE | 0,20 |
| SALCARE^{®} SC 80 | 1,80 |
| EDTA DISODIQUE | 0,10 |
| ACIDE BENZOÏQUE | 0,01 |
| EMULGADE^{®} F | 4,3 |
| ALCOOL ÉTHYLIQUE DÉNATURÉ | 0,99 |
| O-CRÉSOLPHTALÉINE | 0,01 |
| ACIDE PHOSPHORIQUE | 0,06 |
| SOLUTION EAU OXYGÉNÉE 130 VOL | 34,77 |
| D-PANTHÉNOL | 0,2 |
| EAU DÉMINÉRALISÉE | 56,81 |

La proportion massique de la solution d'eau oxygénée 130V dans la composition oxydante est de 34,77 %. La composition oxydante est une composition à 40V en peroxyde d'hydrogène et la composition de décoloration est à 20V en peroxyde d'hydrogène. La constitution de la composition alcaline est donnée au tableau 6 ci-après.

**Tableau 6**

| MATIÈRE PREMIÈRE | Proportion massique, % |
|---|---|
| GOMME XANTHANE | 0,25 |
| SALCARE^{®} SC 80 | 4,50 |
| HYDROXYDE DE SODIUM | 1,00 |
| MONO-ÉTHANOLAMINE | 4,00 |
| EAU DÉMINÉRALISÉE | 90,25 |

La composition de décoloration formée présente une valeur de pH de 10,5. On applique la composition de décoloration sur une zone test pileuse de l'avant-bras de 10 personnes volontaires et on maintient la composition de décoloration au contact de la zone pileuse à décolorer pendant un temps de pause :
- de 5 minutes pour un premier groupe ;
- de 7 minutes pour un deuxième groupe, et
- de 9 minutes pour un troisième groupe.

On retire la composition de décoloration de la zone pileuse par rinçage à l'eau, puis on sèche la zone pileuse par essuyage. On interroge les personnes volontaires et on note un indice de satisfaction (satisfaite à très satisfaite / moyennement satisfaite à assez satisfaite / peu satisfaite à pas satisfaite) procuré par le traitement de décoloration. Les résultats sont donnés dans le tableau 7 ci-après.

**Tableau 7.**

| Temps de pause, min | Appréciation | | |
|---|---|---|---|
| | Satisfait à très satisfait | Moyennement à assez satisfait | Peu à pas satisfait |
| 5 | 67 % | 33 % | 0 % |
| 7 | 100 % | 0 % | 0 % |
| 9 | 100 % | 0 % | 0 % |

Pour une durée de traitement de dépigmentation avec une composition de décoloration sans ammoniaque et sans sel d'ammonium selon l'invention, 100 % des personnes interrogées sont satisfaites de la décoloration obtenue.

On sollicite en outre ces personnes pour attribuer une note concernant l'aspect, la couleur, l'odeur, la facilité d'étalement, la tenue, le temps de pause et l'efficacité globale de la composition selon l'invention. Les résultats sont donnés au tableau 8 ci-après dans lequel la note de 6 traduit une satisfaction maximale et la note de 0 traduit une satisfaction minimale.

**Tableau 8**

| Composition de dépigmentation | Note |
|---|---|
| Aspect | 5 à 6 |
| Couleur | 5 à 6 |
| Odeur | 5 à 6 |
| Facilité à l'étalement | 5 |
| Tenue | 5 à 6 |
| Temps de pause | 4 à 5 |
| Efficacité | 5 |

### EXEMPLE 2 - Essai comparatif

À titre d'essai comparatif, réalise une composition de décoloration comprenant de l'ammoniaque associée à une formulation oxydante d'H₂O₂ 12V, qui ne constitue pas une composition de décoloration selon l'invention. 67 % des personnes bénéficiant d'un tel traitement avec un temps de pause de 5 minutes sont satisfaites à très satisfaites. La composition de décoloration selon l'invention est aussi efficace -notamment pour un temps de pause de 5 minutes-qu'une composition contenant de l'ammoniaque, mais n'en présente pas les inconvénients liés à l'odeur et à la production de rougeurs.

## Revendications

1. Composition sans ammoniaque et sans sel d'ammonium, cosmétiquement acceptable comprenant :
- au moins un agent de décoloration de poils, et ;
- au moins une amine organique choisie à l'exclusion de tout acide α-aminé basique, et ;
- au moins une base forte minérale ;
**caractérisée en ce que** ladite au moins une base forte minérale :
- présente dans la composition une proportion massique comprise entre 0,2 % et 2 %, et ;
- est présente dans la composition en une quantité adaptée pour que le pH de la composition soit compris entre pH 9,0 et pH 12.

2. Composition selon la revendication 1, **caractérisée en ce qu'**au moins une amine organique est choisie dans le groupe formé des amines organiques présentant de 1 à 6 atomes de carbone.

3. Composition selon l'une des revendications 1 ou 2, **caractérisée en ce qu'**au moins une amine organique est choisie dans le groupe formé de la mono-éthanolamine, de la mono-isopropanolamine, de l'éthylamine et de leurs sels, de la guanidine et de ses sels, du bicarbonate d'amino-guanidine et de la tri-éthanolamine.

4. Composition selon l'une des revendications 1 à 3, **caractérisée en ce qu'**au moins une amine organique présente une proportion massique comprise entre 0,1 % et 40 % dans la composition.

5. Composition selon l'une des revendications 1 à 4, **caractérisée en ce qu'**au moins une base forte minérale est choisie dans le groupe formé de l'hydroxyde de sodium (NaOH), de l'hydroxyde de potassium (KOH) de l'hydroxyde de strontium (Sr(OH)₂), de l'hydroxyde de calcium (Ca(OH)₂) et de l'hydroxyde de lithium (LiOH).

6. Procédé de décoloration de poils dans lequel :
- on prépare extemporanément une composition de décoloration sans ammoniaque et sans sel d'ammonium, cosmétiquement acceptable comprenant :
o au moins un agent de décoloration des poils;
o au moins une amine organique choisie à l'exclusion de tout acide α-aminé basique, et ;
o au moins une base forte minérale, puis ;
- on applique ladite composition de décoloration sur les poils à décolorer et on maintient ladite composition de décoloration en contact avec lesdits poils pendant une durée suffisante pour obtenir ladite décoloration ;
**caractérisé en ce que** ladite au moins une base forte minérale :
- présente dans la composition de décoloration une proportion massique comprise entre 0,2 % et 2 %, et ;
- est présente dans la composition de décoloration en une quantité adaptée pour que le pH de la composition de décoloration soit compris entre pH 9,0 et pH 12,0.

7. Procédé selon la revendication 6, **caractérisé en ce qu'**on prépare la composition de décoloration par mélange :
- d'une composition acide oxydante comprenant chaque agent de décoloration, et ;
- d'une composition alcaline comprenant chaque amine organique et chaque base forte minérale et présentant une valeur de pH comprise entre pH 10,0 et pH 13,0.

8. Procédé selon la revendication 7, **caractérisé en ce que** la composition alcaline présente une proportion massique d'amine organique comprise entre 0,2 % et 50 %.

9. Procédé selon l'une des revendications 7 ou 8, **caractérisé en ce que** la composition alcaline comprend une proportion massique totale de base forte minérale comprise entre 0,2 % et 10 %.

10. Procédé selon l'une des revendications 7 à 9, **caractérisé en ce que** pour préparer la composition alcaline, on choisit un ordre d'addition des constituants de ladite composition alcaline dans lequel on introduit la(les) base(s) forte(s) minérale(s) préalablement à l'introduction de l'(des) amine(s) organique(s) en vue de former la composition alcaline.

11. Procédé selon l'une des revendications 7 à 10, **caractérisé en ce que** la composition oxydante comprend au moins un indicateur de mélange choisi dans le groupe formé des indicateurs de pH qui sont colorés à une valeur de pH comprise entre pH 8,0 et pH 12,0 et qui sont incolores à une valeur de pH comprise entre pH 1,0 et pH 8,0 et à une valeur de pH comprise entre pH 12,0 et pH 14,0.

12. Procédé selon l'une des revendications 7 à 11, **caractérisé en ce que** la composition oxydante présente une valeur de pH inférieure à 7.

13. Procédé selon l'une des revendications 7 à 12, **caractérisé en ce qu'**on maintient la composition de décoloration en contact avec les poils pendant une durée inférieure à 15 min.

14. Procédé selon la revendication 7, **caractérisé en ce qu'**au moins l'une des compositions oxydante et alcaline étant sous forme d'un solide à l'état divisé, on forme la composition de décoloration par mélange de la composition oxydante, de la composition alcaline et, le cas échéant, d'une quantité prédéterminée d'au moins un solvant.

15. Nécessaire de décoloration adapté pour préparer une composition selon l'une des revendications 1 à 5, comprenant :
- un premier dispositif contenant une composition oxydante acide sans ammoniaque et sans sel d'ammonium, cosmétiquement acceptable et comprenant au moins un agent de décoloration de poils, et ;
- un deuxième dispositif contenant une composition alcaline aussi sans ammoniaque et sans sel d'ammonium, cosmétiquement acceptable, et comprenant au moins une amine organique et au moins une base forte minérale ;
**caractérisé en ce que** la composition oxydante et la composition alcaline sont exemptes de tout acide α-aminé basique, la composition alcaline présentant une valeur de pH comprise entre pH 10,0 et pH 13,0.
